# EUROPEAN PATENT APPLICATION

(11) **EP 4 317 430 A1**
(43) Date of publication of application: **07.02.2024**
(21) Application number: 22774319.2
(22) Date of filing: 24.03.2022
(51) Int. Cl.: C12N 15/09, A01H 1/00, A01H 5/00, C12N 5/04

(54) **METHOD FOR IMPROVING PLANT GENETIC TRANSFORMATION AND GENE EDITING EFFICIENCY**

(30) Priority: 25.03.2021 CN 202110321591
(71) Applicant: Suzhou Qi Biodesign biotechnology Company Limited, Jiangsu 215127 (CN)
(72) Inventor: GAO, Caixia, Beijing 100101 (CN); CHEN, Lu, Beijing 100101 (CN)
(74) Representative: Nederlandsch Octrooibureau
(86) International application number: PCT/CN2022/082830
(87) International publication number: WO 2022/199665

(57) **Abstract**

The invention belongs to the field of genetic engineering in plants. In particular, the invention relates to a method for improving efficiency of genetic transformation and gene editing in plants. More particularly, the invention relates to a method for improving regeneration efficiency of genetic transformation and/or gene editing efficiency in plants by expression of gene NiR which promotes nitrogen metabolism in plants.

## Description

### Technical Field

The invention belongs to the field of genetic engineering in plants. In particular, the invention relates to a method for improving efficiency of genetic transformation and gene editing in plants. More particularly, the invention relates to a method for improving regeneration efficiency of genetic transformation and/or gene editing efficiency in plants by expression of gene NiR which promotes nitrogen metabolism in plants.

### Technical background

Crop genetics and breeding has experienced artificial selection breeding, cross breeding, mutation breeding and molecular marker-assisted breeding based on molecular technology. With the gradual decrease in genetic diversity of available varieties, traditional breeding shows an increasing bottleneck effect, that is, conventional breeding techniques cannot meet human needs and development of sustainable agriculture due to difficulty in breeding of groundbreaking new varieties. The rapid development of life sciences has moved from "reading" of biogenetic information to the post-genomic era, and precise "rewriting" and even "new design" of genomes are coming true. Such biotechnology, aiming at creating new traits or organisms through design, has shown tremendous potential in for example the fields of treatment of disease, pharmaceuticals, manufacturing, and particularly agriculture.

Genome editing technology is a revolutionary means in current life sciences, and may arrive at precise, efficient, and specific rewriting of genomes, thereby revolutionarily promoting research and exploration in the entire field of life sciences. Genome editing refers to deletion, replacement, insertion, and other operations on targeted genes to rewrite genetic information and thus to obtain new functions or phenotypes, even create new species. Developing efficient and precise breeding techniques suitble for crops based on gene editing technology will overcome the shortcomings of traditional breeding, and arrive at molecular design breeding with precise genome alteration. This is of important strategic significance for the future development of agriculture.

Current genome editing technologies mainly include ZFN (Zinc-fingernuclease), TALEN (Transcription activator-like effector nuclease) and CRISPR (Clustered regularly interspaced short palindromic repeats) /Cas system. The CRISPR/Cas system is a simplest and widely used genome editing technology system due to its high efficiency and flexibility. In the CRISPR/Cas system, Cas protein can target anywhere in a genome under the guidance of an artificial designed guide RNA. Base editing system, a new kind of genome editing technologies developed based on the CRISPR system, are divided into cytosine base editing system and adenine base editing system.

They comprise cytidine deaminase and adenine deaminase, respectively, fused with Cas9 single-stranded nickase that then produces a single-stranded DNA region under targeted effect of the guide RNA, so that the deaminases can efficiently deaminate C- and A-nucleotides at targeted positions of the single-stranded DNA and convert them into U and I bases that are then repaired into T and G bases in the process of cellular autorepair, respectively. The base editing technology overcome the shortcomings of traditional DSB-mediated genome editing and can efficiently achieve precise replacement of a single base. The potent genome modification system mediated by the CRISPR/Cas system will provide a strong technical support for plant genomics research and new plant molecular design breeding, accelerating the breeding of new crop varieties and realizing the sustainable development of agriculture.

A key step in plant gene editing is delivering the gene-editing nuclease proteins or coding nucleic acids thereof to plant cells so as to achieve editing of target gene. Currently, the delivery techniques for plant genome editing are mainly achieved by genetic transformation and tissue culture, including agrobacterium-mediated transformation and particle bombardment. Important progress has been made in plant transformation and genetic modification in recent years. However, transformation and genetic modification of some important agronomic plants, such as octoploid strawberry (Fragaria× ananassa) are difficult and time-consuming.

### Brief Description of the Drawings

Figure 1 shows a vector map used in Example 1 of the present application.
Figure 2 shows that strawberry NiR can significantly improve the seedlings regeneration efficiency from the strawberry callus.
Figure 3 shows a vector map used in Example 2 of the present application.
Figure 4 shows that wheat NiR can significantly improve the regeneration efficiency from wheat callus.
Figure 5 shows a vector map used in Example 4 of the present application.
Figure 6 shows that tomato NiR can significantly improve the regeneration efficiency of tomato.

### Specific embodiments

### I. Definitions

In the present invention, unless otherwise specified, scientific and technical terms used in this text have meanings as generally understood by those skilled in the art. In addition, terms related to protein and nucleic acid chemistry, molecular biology, cell and tissue culture, microbiology and immunology as well as laboratory procedures used in this text all correspond to terms and standard procedures which are widely employed in their respective fields. For example, standard recombinant DNAs and molecular cloning techniques used in the present invention are well known to those skilled in the art and more comprehensively described in the following literature: Sambrook, J., Fritsch, E.F. and Maniatis, T., Molecular Cloning: A Laboratory Manual; Cold Spring Harbor Laboratory Press: Cold Spring Harbor, 1989 (hereinafter referred to as "Sambrook"). Meanwhile, definitions and explanations of related terms are provided below in order to further clarify the invention.

As used herein, the term "and/or" encompasses any combinations of the items connected by this term, equivalent to listing all the combinations individually. For example, "A and/or B" encompasses "A", "A and B" and "B". For another example, "A, B and/or C" encompasses "A", "B", "C", "A and B", "A and C", "B and C" and "A and B and C".

When the term "comprise" is used herein to describe sequences of proteins or nucleic acids, the proteins or nucleic acids may be composed of the sequences, or provided with additional amino acids or nucleotides at one or both ends thereof, but still have the activity described in the present invention. In addition, those skilled in the art are aware that methionine encoded by an initiation codon at N-terminal of polypeptide are retained in some practical cases (such as when expressed in a specific expression system), but does not substantially affect the function of the polypeptide. Therefore, when a specific polypeptide amino acid sequence is described in the specification and claims of the present application, although it may not contain methionine encoded by the initiation codon at N-terminal, sequences containing the methionine is also encompassed. Accordingly, its coding nucleotide sequences may also contain the initiation codon, and vice versa.

"Genome" as used herein encompasses not only the chromosomal DNA in the nucleus, but also the organelle DNA in the subcellular components of the cells, such as mitochondria and plastids. "Exogenous" with respect to sequence means a sequence that originates from a foreign species, or, if from the same species, is substantially modified from its native form in composition and/or genomic locus by deliberate human intervention.

"Nucleic acid sequences", "polynucleotides", "nucleotide sequences" or "nucleic acid fragments" are used interchangeably and is a polymer of RNA or DNA that is single-or double-stranded, optionally containing synthetic, non-natural or altered nucleotide bases. Nucleotides are referred to by their single letter designation as follows: "A" for adenylate or deoxyadenylate (for RNA or DNA, respectively) , "C" for cytidylate or deoxycytidylate, "G" for guanylate or deoxyguanylate, "U" for uridylate, "T" for deoxythymidylate, "R" for purines (A or G) , "Y" for pyrimidines (C or T) , "K" for G or T, "H" for A or C or T, "I" for inosine, and "N" for any nucleotide.

"Polypeptides", "peptides", and "proteins" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptides", "peptides", "amino acid sequences" and "proteins" are also inclusive of modifications including, but not limited to, glycosylation, lipid attachment, sulfation, gamma-carboxylation of glutamic acid residues, hydroxylation and ADP-ribosylation.

As used in the invention, an "expression construct" refers to a vector, such as a recombinant vector, which is suitable for the expression of a nucleic acid sequence of interest in organisms. "Expression" refers to the production of a functional product. For example, expression of a nucleic acid fragment may refer to transcription of the nucleic acid fragment (e.g., transcription resulting in mRNA or functional RNA) and/or translation of mRNA into a precursor or mature protein.

The "expression construct" of the invention may be linear nucleic acid fragment (including DNA or RNA fragment), circular plasmid, and viral vector.

The "expression construct" of the present invention may include regulatory sequences and nucleic acid sequences of interest operably linked thereto. Regulatory sequences and nucleic acid sequences of interest can be derived from different sources, or can be derived from the same source, but arranged in a manner different than that normally found in nature.

"Regulatory sequences" and "regulatory elements" are used interchangeably and refer to nucleotide sequences located upstream (5' non-coding sequences) , within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences. "Promoter" refers to a nucleic acid fragment capable of controlling transcription of another nucleic acid fragment. In some embodiments of the invention, a promoter is one capable of controlling gene transcription in cells, regardless of whether it is derived from the cells or not. The promoter can be constitutive, tissue-specific, development-regulatory or inducible.

As used herein, the terms "operatively linked" and "operably linked" refer to regulatory elements (such as, but not limited to promoter sequences, transcription terminator sequences, etc.) that are connected to a nucleotide sequence (for example, a coding sequence or open reading frame) in such a way that the transcription of the nucleotide sequence is controlled and regulated by that transcriptional regulatory element. Techniques for operatively linking a promoter region to a nucleotide sequence are known in the art.

"Introducing" a nucleic acid molecule (such as an expression construct) into plant cells means presenting to the plant cell the nucleic acid molecule such a manner that the sequence gains access to the interior of a plant cell.

"Regeneration" refers to the process of growing an intact plant from a plant cell or cells (e.g., plant protoplast, callus, or explant).

### II. Improvement of plant regeneration, transformation and gene editing

In one aspect, the invention provides a method for improving regeneration efficiency of a plant cell, comprising, a) overexpressing NiR in the plant cell, such as introducing an expression construct containing a coding nucleic acid sequence of NiR into the plant cell. In some embodiments, the method also comprises, b) regenerating into an intact plant from the plant cell obtained from a).

In another aspect, the invention provides a method for improving efficiency of transforming exogenous nucleic acid sequences of interest in a plants or for transforming exogenous nucleic acid sequences of interest into a plant. The method comprises:
(a) overexpressing NiR in a cell of the plant, such as introducing an expression construct containing a coding nucleic acid sequence of NiR into the plant cell;
(b) introducing at least one expression construct containing at least one exogenous nucleic acid sequence of interest into the plant cell;
(c) regenerating into an intact plant from the plant cell.

In some embodiments, steps (a) and (b) are performed in parallel (simultaneously). In some embodiments, step (a) is performed before step (b). In some embodiments, step (b) is performed before step (a).

In another aspect, the invention provides a method for improving efficiency of gene editing in plants or for gene editing in plants. The method includes:
(a) overexpressing NiR in a cell of the plant, such as introducing an expression construct containing a coding nucleic acid sequence of NiR into the plant cell;
(b) introducing at least one expression construct containing at least one exogenous sequence of interest into the plant cells, wherein the at least one exogenous sequence of interest encodes a component of gene editing system;
(c) regenerating into an intact plant from the plant cell.

In some embodiments, steps (a) and (b) are performed in parallel (simultaneously). In some embodiments, step (a) is performed before step (b). In some embodiments, step (b) is performed before step (a).

In some embodiments of the method of the invention, the overexpression of NiR in the plant cell can also be achieved by modifying expression regulatory sequence of the endogenous NiR gene in the plant cell. For example, overexpression of NiR in the plant cell can be achieved by genetically editing the expression regulatory sequence of the endogenous NiR gene in the plant cell. Therefore, in some embodiments, the method includes introducing a gene editing system for the expression regulatory sequence of the endogenous NiR gene into the plant cell (including introducing one or more expression constructs encoding the component of the gene editing system), resulting in overexpression of the endogenous NiR gene in the plant cell.

In some embodiments of the method of the invention, overexpression of NiR in the plant cell also includes increasing bioactivity of the NiR protein endogenously expressed in the plant cell. In some embodiments, mutation that causes increase of the bioactivity can be introduced into the endogenous NiR protein, for example, by gene editing. Therefore, in some embodiments, the method includes introducing a gene editing system for the endogenous NiR coding sequence into the plant cell (such as introducing one or more expression constructs encoding the component of the gene editing system), resulting in increase of the bioactivity of the NiR protein endogenously expressed in the plant cell.

The invention also provides a kit for performing the method of the invention. The kit includes at least an expression construct containing a coding nucleic acid sequence of NiR, or one or more expression constructs encoding the component of the gene editing system, wherein the gene editing system targets the expression regulatory sequence or coding sequence of the endogenous NiR, and is capable of causing overexpression of the endogenous NiR gene in the plant cell or increase of the bioactivity of the NiR protein endogenously expressed in the plant cell.

The present invention also provides an expression construct containing a coding nucleic acid sequence of NiR, or one or more expression constructs encoding the component of the gene editing system targeting the expression regulatory sequence or coding sequence of the endogenous NiR, for use in improving the regeneration efficiency of the plant cell in plant transformation, improving the efficiency of transforming an exogenous nucleic acid sequence of interest in the plant or improving the efficiency of gene editing in the plant, wherein the gene editing system targeting the expression regulatory sequence or coding sequence of the endogenous NiR can cause overexpression of the endogenous NiR gene in the plant cell or increase of the bioactivity of the NiR protein endogenously expressed in the plant cell.

Nitrite reductase (NiR) is an enzyme that widely exists in microorganisms and plants and catalyzes the reduction of nitrite. The nitrite reductase (NiR) is responsible for reducing nitrite into ammonium in plants, and serves as an important enzyme in the process of nitrogen assimilation in plants. The inventor surprisingly found that overexpression of NiR in plant cells can significantly improve the efficiency of regenerating into intact plants from the plant cells as well as the efficiency of transforming exogenous nucleic acid sequences of interest into plants. More surprisingly, the efficiency of gene editing can be improved when the exogenous nucleic acid sequences of interest encode the gene editing system.

In some embodiments, the NiR is the NiR derived from a plant or functional variant thereof, such as the NiR derived from wheat, strawberry, rice, corn, soybean, sunflower, sorghum, rape, alfalfa, cotton, barley, millet, sugarcane, tomato, tobacco, cassava, potato or arabidopsis or functional variant thereof. In some embodiments, the NiR is the NiR derived from a plant species to be regenerated, transformed or genetically edited or functional variant thereof. In some embodiments, the NiR is the NiR derived from a plant species different from the plant species to be regenerated, transformed or genetically edited or functional variant thereof.

In some embodiments, the NiR is a strawberry NiR or functional variant thereof. The exemplary strawberry NiR or functional variant thereof, for example, contains an amino acid sequence shown in SEQ ID NO:1, or an amino acid sequence having at least 75%, 80%, 85%, 90%, 95%, 96%, 97%, 98%, and 99% identity to SEQ ID NO:1.

In some embodiments, the NiR is a wheat NiR or functional variant thereof. The exemplary wheat NiR or functional variant thereof, for example, contains an amino acid sequence shown in SEQ ID NO:3, or an amino acid sequence having at least 75%, 80%, 85%, 90%, 95%, 96%, 97%, 98%, and 99% identity to SEQ ID NO:3.

In some embodiments, the NiR is a tomato NiR or functional variant thereof. The exemplary tomato NiR or functional variant thereof, for example, contains an amino acid sequence shown in SEQ ID NO:5, or an amino acid sequence having at least 75%, 80%, 85%, 90%, 95%, 96%, 97%, 98%, and 99% identity to SEQ ID NO:5.

In some embodiments, the coding nucleic acid sequence of the NiR and the at least one exogenous nucleic acid sequence of interest are in the same expression construct. In some embodiments, the coding nucleic acid sequence of the NiR and the at least one exogenous nucleic acid sequence of interest are in different expression constructs, respectively. In some embodiments, the coding nucleic acid sequence of the NiR is in one expression construct while the at least one exogenous nucleic acid sequence of interest is in another expression construct.

In some embodiments, the coding nucleic acid sequence of the NiR and/or the at least one exogenous nucleic acid sequence of interest are operably linked to a transcriptional regulatory element.

The "at least one exogenous nucleic acid sequence of interest" may be any nucleic acid sequence that need to be transformed into a plant. For example, the exogenous nucleic acid sequence of interest may be a nucleic acid sequence encoding a trait critical for agriculture, insect resistance, disease resistance, herbicide resistance, sterility and commercial products. The nucleic acid sequence of interest may also include that involved in the metabolism of oil, starch, carbohydrates or nutrients, as well as those that affect fruit size, sucrose load, etc.

In some preferred embodiments, the "at least one exogenous nucleic acid sequence of interest" encodes the component of the gene editing system so that the plant can be genetically edited. The gene editing system can target an endogenous gene of a trait that is critical for agriculture, insect resistance, disease resistance, herbicide resistance, sterility and commercial products, or expression regulatory sequence thereof, thereby realizing the modification on the expression or activity of the endogenous genes. For example, the gene editing system can target an endogenous gene involved in the metabolism of oil, starch, carbohydrates or nutrients, or expression regulatory sequence thereof, or an endogenous gene that affects fruit size, sucrose load, etc., or expression regulatory sequence thereof, thereby realizing the modification on the expression or activity of the endogenous gene.

"Gene editing", also known as genome editing, refers to a process in which nucleotide is inserted, deleted or replaced in the genome of an organism with sequence-specific nucleases or derivatives thereof. The gene editing usually creates site-specific double strand breaks (DSBs) at desired locations in the genome, followed by introducing insertion, deletion or replacement of desired DNAs in the process of DSB repair. However, gene editing can also encompass base editing techniques, transcription activation or inhibition, and epigenetic modification that does not involve DSB, as long as it is sequence specific.

The gene editing systems used in the invention is not particularly limited. For example, the gene editing system useful in the present invention includes, but not limited to, zinc-fingernuclease (ZFN), meganuclease (MGN), transcription activator-like effector nuclease (TALEN), and CRISPR (clustered regularly interspaced short palindromic repeats) system.

The "zinc-fingernuclease (ZFN)" is an artificial restriction enzyme, which is generated by combining a zinc finger DNA-binding domain with a DNA-cleavage domain. The DNA-binding domains of individual ZFNs typically contain 3-6 individual zinc finger repeats and can each recognize e.g. 3bp specific sequence. By combining different zinc finger repeats, different genome sequences can be targeted.

The meganuclease usually refers to a homing endonuclease that can recognize 14-40bp nucleic acid sequences. The meganuclease has high specificity due to the long recognized sequences, thus reducing off-target effect.

Transcription activator-like effector nucleases (TALEN) are restriction enzymes that can be engineered to cut specific sequences of DNA. They are usually generated by combining a transcription activator-like effectors DNA-binding domain with a DNA cleavage domain. TALE can be engineered to bind almost any desired DNA sequences.

The "CRISPR system" usually contains two components that can form sequence-specific complexes: CRISPR nuclease or variant thereof, and corresponding guide RNA. Therefore, with respect to a CRISPR system, the "at least one exogenous nucleic acid sequence of interest" of the present invention may include a coding nucleic acid sequence of the CRISPR nuclease or variant thereof, and/or a coding nucleic acid sequence of the corresponding guide RNA.

In some preferred embodiments, the gene editing system is a CRISPR system. A variety of different CRISPR gene editing systems known in the art can be used in the invention. For example, suitable CRISPR gene editing systems can be found at http://www.adducnc.oru/crispr/. The CRISPR gene editing system includes that modifies genome sequence and that is used for transcriptional regulation without modifying genome sequences.

A "CRISPR nuclease", as used herein, is any nuclease which has been identified in a naturally occurring CRISPR system. "CRISPR nuclease variant" includes modified forms, artificial mutants (including nickase mutants), catalytically active fragments of the natural CRISPR nuclease or its fusions with other functional proteins/peptides. A variety of artificial functional variants of the CRISPR nuclease are known in the art, such as highly specific variants or nickase variants, or its fusion proteins with cytidine deaminase or adenosine deaminase, etc. The CRISPR nuclease or variant thereof can recognize, bind, and/or cleave target nucleic acid structure by interacting with the corresponding guide RNA. Those skilled in the art know how to select a suitable CRISPR nuclease or variant thereof to achieve the purpose of the present invention.

The CRISPR nuclease or variant thereof used in the CRISPR gene editing system of the present invention may, for example, be selected from Cas3, Cas8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Cas9, Csn2, Cas4, Cpfl (Cas12a), C2c1, C2c3 or C2c2 proteins, or functional variants of these nucleases.

In some embodiments, the CRISPR nuclease or variant thereof includes a Cas9 nuclease or variant thereof. The CRISPR gene editing system based on the Cas9 nuclease or variant thereof is also called CRISPR-Cas9 gene editing system herein. The Cas9 nuclease may be a Cas9 nuclease derived from different species, such as spCas9 from *Streptococcus pyogenes* (*S. pyogenes*).

The Cas9 nuclease variant may include Cas9 nickase (nCas9), in which one of the two subdomains (HNH nuclease subdomain and RuvC subdomain) in a DNA cleavage domain of the Cas9 nuclease is inactivated to form nickase. In some embodiments, the Cas9 nickase can be used to combine with two gRNAs targeting the upstream and downstream of a sequence to be edited, thereby achieving deletion of the sequence to be edited, or replacement of the sequence to be edited in the presence of a donor sequence.

In some embodiments, the CRISPR nuclease or variant thereof may also include Cpfl (Cas12a) nuclease or variant thereof, such as highly specific variants. The Cpfl nuclease may be a Cpfl nuclease derived from different species, such as Cpfl nuclease from *Francisella novicida* U112, *Acidaminococcus sp.* BV3L6 and *Lachnospiraceae bacterium* ND2006. The CRISPR gene editing system based on the Cpfl nuclease or variant thereof is also called CRISPR-Cpfl system herein. In some embodiments, the CRISPR nuclease variant may also include base editor. The base editor usually is a fusion protein of CRISPR nuclease variant without DNA cleavage activity and deaminase.

As used in the present invention, the "CRISPR nuclease variant without DNA cleavage activity" includes, but not limited to, Cas9 nickase (nCas9), nuclease dead Cas9 (dCas9), or nuclease dead Cpfl (dCpf1). The nuclease dead Cas9 (dCas9) or the nuclease dead Cpfl (dCpf1) are complete absence of DNA cleavage activity. A variety of DNA nuclease variants without CRISPR cleavage activity are known in the art.

As used in the invention, the "deaminase" refers to an enzyme that catalyzes deamination. In some embodiments of the invention, the deaminase refers to cytidine deaminase capable of using single-stranded DNA as a substrate and catalyzing deamination of cytidine or deoxycytidine to uridine or deoxyuridine respectively. In some embodiments of the invention, the deaminase refers to adenine deaminase capable of using single-stranded DNA as a substrate and catalyzing adenosine or deoxyadenosine (A) to form inosine (I). A variety of suitable cytidine deaminases or adenine deaminases using single-stranded DNA as a substrate are known in the art. Suitable cytidine deaminase includes, but not limited to, APOBEC1 deaminase, activation-induced cytidine deaminase (AID), APOBEC3G, CDA1, and human APOBEC3A deaminase. In some preferred embodiments, the cytidine deaminase is human APOBEC3A. Examples of suitable adenine deaminases include, but not limited to, DNA-dependent adenine deaminase disclosed by Nicloe M. Gaudelli et al. (doi:10.1038/nature24644, 2017).

By fusing the CRISPR nuclease variant without DNA cleavage activity with deaminase (to form a so-called "base editor"), base editing in target nucleotide sequence can be achieved, such as C-to-T conversion or A-to-G conversion. A variety of base editors are known in the art, and those skilled in the art know how to select a suitable base editor to achieve the purpose of the present invention. The CRISPR gene editing system based on base editor is also called a base editing system.

As used herein, "guide RNA" and "gRNA" are used interchangeably and refer to a RNA molecule that can form a complex with the CRISPR nuclease or variant thereof and target the complex to a target sequence due to a certain identity with the target sequence. For example, the gRNA used by the Cas9 nuclease or variant thereof usually consists of crRNA and tracrRNA molecules that partially complement with each other to form a complex, wherein the crRNA contains guide sequence having sufficient identity with the target sequence to hybridize with complementary strand of the target sequence and capable of directing the CRISPR complex (Cas9 + crRNA + tracrRNA) to specifically bind to the target sequence. However, it is known in the art that a single-guide RNA (sgRNA) containing the characteristics of both crRNA and tracrRNA may be designed. The gRNA used by Cpfl nuclease or variant thereof is usually only composed of mature crRNA molecule, and also called sgRNA. Designing a suitable gRNA on the basis of the CRISPR nuclease or variant thereof and target sequence to be edited is within the ability of those skilled in the art.

The sequence-specific nucleases used in the gene editing in the invention, such as zinc-fingemuclease, transcription activator-like effector nuclease or CRISPR nuclease or variant thereof, can also include subcellular localization signal (e.g., nuclear localization signal), peptide linker, detectable tag and other elements. For example, the base editor in the CRISPR base editing system usually contains one or more nuclear location signals (NLS), facilitating its access to nucleus and achieving the editing of the chromosome DNA.

The expression construct of the present invention can be introduced into the plant cell by one of a variety of methods known in the art, including, but not limited to, particle bombardment, PEG-mediated protoplast transformation and agrobacterium-mediated transformation.

In some embodiments, the plant cell of the present invention is the cell suitable for regenerating into an intact plant by tissue culture. Examples of suitable plant cell include, but not limited to, protoplast cells, callus cells, immature embryo cells, and explant cells.

Methods of regenerating into a transformed intact plant by culturing transformed protoplast, callus, immature embryo or explant are known in the art. In the process of regeneration, transformants can also be screened based on the selective marker carried on the introduced expression construct. In some embodiments, the regeneration is performed in the absence of selection pressure.

In some embodiments, the expression construct of the present invention is transiently transformed into the plant cell. Transient transformation refers to that the construct is introduced into cells to take effect but not integrated into the genome of the cells. This is particularly useful for gene editing because it can generate non-transgenic modified plants.

The plant suitable for being transformed or genetically edited by the method of the invention may be a monocotyledonous plant or a dicotyledonous plant. For example, examples of the plant include, but not limited to, wheat, strawberry, rice, corn, soybean, sunflower, sorghum, rape, alfalfa, cotton, barley, millet, sugarcane, tomato, tobacco, cassava and potato. The method of the invention is particularly suitable for genetic transformation or gene editing on plant varieties or genotypes that were previously difficult to be transformed. In some specific embodiments, the plant is wheat, for example, the wheat is Xiaoyan 54 or Jimai 22. In some specific embodiments, the plant is strawberry, for example, the strawberry may be different varieties of strawberry plants, such as Benihoppe and Tochiotome.

In some embodiments of the present invention, the coding nucleic acid sequence or nucleic acid sequence of interest is codon optimized for plant species in order to obtain effective expression in the plant.

Codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at kazusa.orjp/codon/, and these tables can be adapted in a number of ways. See Nakamura, Y, et al. "Codon usage tabulated from the international DNA sequence databases: status for the year 2000" Nucl. Acids Res. 28:292 (2000).

In some embodiments, the coding nucleic acid sequence of NiR is selected from SEQ ID NO:2, 4 or 6.

In one aspect, the present invention provides the plant and progeny thereof obtained by the method of the present invention.

### Examples

The invention can be further understood by referring to some specific examples given herein. These examples are used only to illustrate the invention and not intended to limit the scope of the invention. It is clear that a variety of modifications and changes can be made to the invention without departing from the essence of the invention, and therefore these modifications and changes are also within the scope of protection of the present application.

### Example 1. Increasing the efficiency of genetic transformation and gene editing in strawberry by overexpression of nitrite reductase NiR

### 1. Vector construction

Nitrite reductase in rice can improve regeneration efficiency of the dominant varieties. In this study, octoploid strawberry NiR was selected to study whether it could improve the genetic transformation and gene editing in strawberry by detection of different constructions.

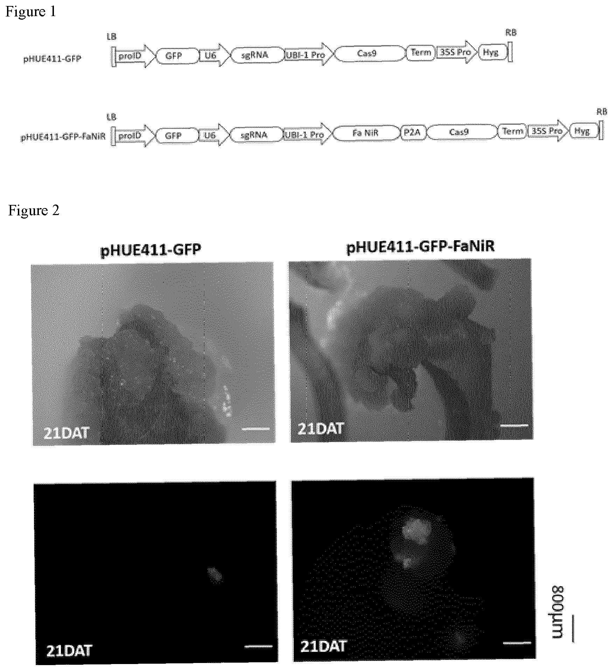

First, a target gene FaNiR highly homologous to rice NiR (amino acid sequence as shown in SEQ ID NO:1, and nucleotide sequence as shown in SEQ ID NO:2), was identified by BLAST. Then, the coding sequences of FaNiR was cloned into a gene editing vector pHUE411-GFP under the control of a ZmUBI promoter, in which the FaNiR and Cas9 were seperately expressed by P2A. The obtained vector was named pHUE411-GFP-FaNiR. The vector map is shown in Figure 1.

### 2. Transformation of strawberry plants

The above constructed vectors were transformed into strawberry callus with agrobacterium. The result of strawberry seedlings regenerated from the callus is shown in Figure 2. It can be seen that overexpression of strawberry endogenous NiR can significantly improve efficiency of regenerating into strawberry seedlings from callus.

### Example 2. Increasing the efficiency of genetic transformation and gene editing in wheat by overexpression of nitrite reductase NiR

In this example, wheat NiR was selected to study whether it could improve the genetic transformation and gene editing in wheat.

### 1. Vector construction

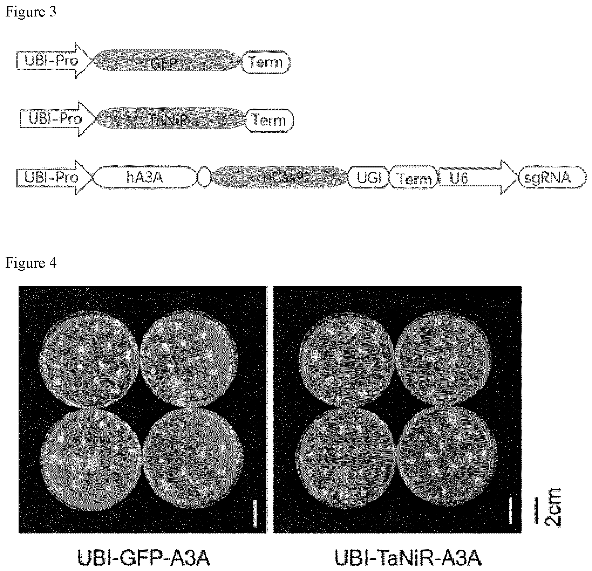

First, a wheat NiR sequence which is most homologous to NiR of easy-to-regenerate Indica rice Kasalath was identified by sequence alignment in NCBI, and then ZmUBI-driven expression was performed after optimization. The constructs are shown in Figure 3.

### 2. Analysis of transformation and editing efficiency by transforming immature wheat embryos through particle bombardment

The above constructed vectors were transformed by wheat particle bombardment system, in which UBI-A3A were co-transformed with TaU6-sgRNA and different booster expression vectors (GFP as control). The wheat ALS gene was selected as the editing site. Transformants were regenerated into plants by tissue culture. Then, the plants were first screened with herbicide-resistant screening, the mutants were further detected with PCR/RE method in the resistant green seedlings. The results are shown in Figure 4 and the table below.

| Plasmids for particle bombardment | Number of bombardments | Total number of embryos | Number of regenerated seedlings | Number of mutant seedlings |
|---|---|---|---|---|
| UBI-GFP-A3A | 5 | 690 | 129 | 5 |
| UBI-TaNiR-A3A | 5 | 601 | 269 | 15 |

The above results showed that UBI-NiR-A3A with integrateion of wheat NiR could improve the regeneration efficiency of wheat transformants by 2.08 times compared to the control UBI-GFP-A3A. Surprisingly, the number of obtained mutants increased significantly, up to 3 times as that of the control, which was much higher than the improvement of regeneration efficiency. This indicates that nitrite reductase NiR, a plant nitrogen metabolism regulator, could be used as an important regulatory protein to promote the efficiency of plant genetic transformation and, more importantly, to improve the efficiency of genome editing.

### Example 3. Increasing the agrobacterium-mediated regeneration efficiency and transformation efficiency in tomato with NiR

### 1. Construction of tomato SlNiR vectors

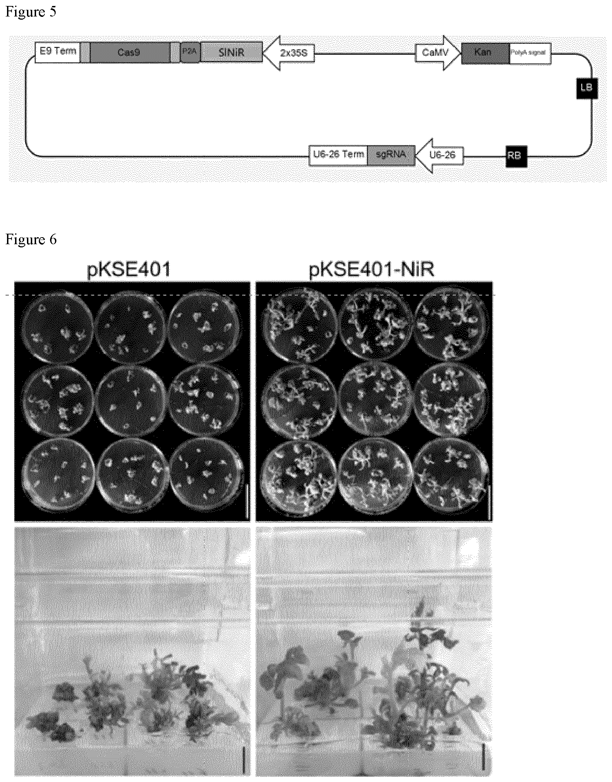

These above results indicates that plant growth factor NiR could increase the regeneration efficiency of tissue culture and gene editing efficiency in monocotyledonous wheat. It cannot determined whether the plant NiR can increase regeneration efficiency in tissue culture of dicotyledons. The inventor selected an endogenous NiR gene of dicotyledonous tomato to study whether it could improve the plant regeneration efficiency in tomato. Tomato SlNiR is selected as the test subject. SlNiR and Cas9 are driven by 35s promoter, and seperated and linked with P2A. sgRNA targeting SlSP1 gene is driven by U6-26 promoter. The construct is shown in Figure 5 (pKSE401-NiR).

### 2. Improvement on regeneration efficiency from tomato callus with tomato NiR

The above constructed vectors were transformed into tomato by agrobacterium. The results regarding the tomato regeneration efficiency are shown in Figure 6 and the table below.

| Treatment | Number of explants | Number of regenerated shoots | Number of transgenic shoots | Number of mutants |
|---|---|---|---|---|
| pKSE401(CK) | 84 | 73 | 30 | 1 |
| pKSE401-NiR | 83 | 108 | 86 | 6 |

The above results indicate that the tomato endogenous NiR could significantly increase the efficiency of regerating into tomato seedlings from callus. In addition, the efficiency of gene editing on the endogenous genes in regenerated plants was also detected and was significantly improved.

### Partial sequences involved in the text

SEQ ID NO:1 Amino acid sequence of strawberry NiR 
MSSFGFLSPPPISSSAPTRQLSAATTPAAAPIVAPDVAAERLEPRVEERQGYWVLKEKFRQGI
NVQEKVKIQREPMKLYMEGGIEELAKLPFEELDKAKDTKDDIDVRLKWLGLFHRRKHHY
GRFMMRLKLPNGVTTSAQTRYLASVIRRYGKDGCADVTTRQNWQIRGIVLADVPDILKG
LAGVGLTSLQSGMDNVRNPVGNPLAGIDPHEIVDTRPYTNLLSQYITGNSLGNPTITNLPR
KWNVCVIGSHDLYEHPHINDLAYMPATKDGKFGFNLLVGGFFSPKRCAEAVPLDAWVSTE
DMIPVCKAILEAYRDLGFRGNRQKTRMMWLIDELGIEGFRSEVVKRMPFKELERASTEDL
VQKQWERRDYIGVHPQKQEGLSFVGLHIPVGRIQADDMDELARIADEYGTGELRLTVEQ
NIIIPNVENTKLQALLQEPLLKEKFSPEPSILMKGLVACTGNQFCGQAIIETKQRALKVTEEV
ERHVSVTRPVRMHWTGCPNTCGQVQVADIGFMGCMARDENGKPVEGVDVFLGGRIGSD
SHLGDIYKKSVPCKDLVPLVIDILVNHFGAVPREREEGEE
SEQ ID NO:2 Coding sequence of strawberry NiR 
ATGTCGTCATTTGGGTTCCTATCTCCTCCTCCTATATCCTCCTCGGCTCCCACGAGGCAG
CTCTCCGCAGCCACTACTCCGGCTGCGGCACCGATTGTCGCTCCGGACGTGGCTGCGG
AGAGGCTGGAGCCGAGGGTGGAGGAGAGACAAGGGTATTGGGTGTTGAAGGAGAAG
TTTCGACAAGGGATTAATGTTCAGGAGAAGGTCAAGATTCAGAGGGAGCCTATGAAGC
TATATATGGAAGGCGGGATTGAAGAGCTTGCTAAGTTGCCTTTTGAGGAGCTTGACAAA
GCTAAGGATACTAAGGATGATATTGATGTTAGGCTCAAGTGGCTTGGTCTCTTCCACAG
AAGAAAGCATCACTATGGTAGATTTATGATGAGGCTGAAGCTTCCGAATGGGGTCACA
ACAAGTGCACAGACAAGATATCTAGCGAGTGTGATACGGAGATATGGCAAGGATGGTT
GTGCAGATGTGACAACCAGGCAAAATTGGCAAATCCGCGGAATTGTGCTGGCAGATGT
GCCTGATATTCTGAAGGGTCTTGCTGGAGTAGGATTGACTAGTCTGCAGAGTGGGATG
GACAATGTGAGAAACCCAGTTGGGAATCCACTTGCAGGCATTGACCCGCATGAAATTG
TTGACACAAGACCTTACACGAACTTGTTGTCCCAATACATTACTGGCAATTCACTCGGC
AATCCAACTATAACAAACTTGCCAAGGAAGTGGAATGTGTGTGTGATAGGGTCTCATGA
TCTTTATGAGCATCCCCACATCAATGATCTTGCGTACATGCCTGCAACAAAGGATGGGA
AATTTGGATTCAATTTACTCGTTGGAGGCTTCTTCAGTCCCAAAAGATGTGCAGAGGCA
GTTCCTCTTGATGCATGGGTCTCAACTGAAGATATGATCCCAGTTTGCAAAGCTATTCTA
GAGGCTTATAGAGACCTTGGATTCAGAGGCAACCGACAAAAGACAAGAATGATGTGGT
TGATCGATGAACTAGGCATAGAAGGATTCAGATCAGAGGTTGTTAAGAGAATGCCCTTT
AAGGAACTGGAAAGAGCATCTACTGAAGACTTGGTACAGAAGCAATGGGAAAGGAGA
GACTATATAGGTGTCCATCCACAGAAACAAGAAGGTCTAAGCTTTGTGGGTCTTCACAT
TCCCGTAGGCAGGATCCAAGCAGATGACATGGATGAGCTAGCTCGTATAGCTGACGAG
TATGGCACTGGGGAACTCCGTCTCACTGTGGAGCAAAACATTATTATCCCCAATGTTGA
GAACACGAAACTCCAAGCATTGCTTCAAGAACCGCTTCTGAAAGAAAAATTTTCACCT
GAACCTTCCATTCTCATGAAAGGACTAGTAGCTTGCACTGGCAACCAGTTTTGCGGCC
AAGCCATCATCGAGACAAAGCAACGGGCCTTGAAGGTGACTGAGGAGGTGGAAAGGC
ATGTGTCAGTGACTCGGCCAGTGAGGATGCATTGGACGGGGTGCCCCAATACCTGCGG
GCAGGTGCAAGTGGCAGATATAGGTTTCATGGGGTGTATGGCAAGGGATGAGAATGGA
AAGCCTGTTGAAGGGGTTGATGTCTTCTTGGGAGGAAGAATAGGAAGTGACTCTCATT
TGGGAGATATTTATAAGAAGAGTGTCCCTTGTAAGGACTTGGTGCCCTTAGTTATTGAC
ATCTTGGTAAATCACTTTGGGGCTGTCCCAAGGGAGAGGGAAGAAGGGGAAGAGTAA
SEQ ID NO:3 Amino acid sequence of wheat NiR 
MALFTEGGIKELAKLPMEQIDADKLTKEDVDVRLKWLGLFHRRKQQYGRFMMRLKLPN
GVTTSEQTRYLAGVIEKYGKEGCADVTTRQNWQIRGVTLPDVPEILEGLRSVGLTSLQSG
MDNVRNPVGSPLAGIDPLEIVDTRPYTNLLSSYITNNSEGNLAITNLPRKWNVCVIGTHDL
YEHPHINDLAYMPAEKDGKFGFNLLVGGFISPKRWGEALPLDAWVPGDDIIPVCKAVLEAF
 
RDLGTRGNRQKTRMMWLIDELGMEAFRSEIEKRMPNGVLERAAAEDLIDKKWERRDYL
GVHPQKQEGLSFVGLHVPVGRLQAADMFELARLADEYGSGELRLTVEQNIVLPNVKNEK
VEALLAEPLLQKFSAHPSLLMKGLVACTGNQFCGQAIIETKARALQVTRDVEARVSVPRA
VRMHWTGCPNSCAQVQVADIGFMGCLTKNSSGKIVEAADIFVGGRVGSDSHLTGVYKKA
VPCEDLVPLVADLLVERFGAVPREREEDEE
SEQ ID NO:4 Coding sequence of wheat NiR 
ATGGCGCTCTTCACGGAGGGCGGCATCAAGGAGCTCGCCAAGCTGCCCATGGAGCAG
ATCGACGCCGACAAGCTCACCAAGGAGGACGTCGACGTGCGGCTCAAGTGGCTCGGC
CTCTTCCACCGCCGCAAGCAGCAGTATGGGCGGTTCATGATGCGGCTGAAGCTGCCCA
ACGGCGTGACGACGAGCGAGCAGACGCGCTACCTGGCCGGCGTGATCGAGAAGTACG
GCAAGGAGGGGTGCGCCGACGTGACGACCCGGCAGAACTGGCAGATCCGCGGCGTG
ACGCTGCCGGACGTGCCGGAGATCCTGGAGGGGCTCCGCTCCGTTGGCCTCACCAGCC
TGCAGAGCGGCATGGACAACGTGCGCAACCCCGTCGGCAGCCCGCTCGCCGGCATCG
ACCCCCTCGAGATCGTCGACACGCGCCCCTACACCAACCTCCTCTCCTCCTACATCACC
AACAACTCCGAGGGCAACCTCGCCATCACCAACCTTCCTAGGAAGTGGAACGTGTGC
GTGATCGGTACCCATGACCTGTACGAGCACCCGCACATCAACGACCTGGCGTACATGC
CGGCCGAGAAGGACGGCAAGTTCGGGTTCAACCTGCTGGTGGGCGGGTTCATCAGCC
CCAAGAGGTGGGGCGAGGCCCTGCCGCTCGACGCCTGGGTCCCCGGCGACGACATCA
TCCCGGTCTGCAAGGCCGTCCTCGAGGCTTTCCGCGACCTCGGCACCAGGGGCAACC
GCCAGAAGACGCGCATGATGTGGCTCATCGACGAGCTCGGGATGGAGGCGTTCCGGTC
GGAGATCGAGAAGAGGATGCCGAACGGCGTGCTGGAGCGCGCGGCGGCCGAGGACC
TGATCGACAAGAAGTGGGAGCGGCGCGACTACCTCGGCGTGCACCCGCAGAAGCAGG
AGGGGCTCTCCTTCGTGGGCCTGCACGTGCCCGTCGGCCGGCTGCAGGCCGCCGACAT
GTTCGAGCTGGCCCGCCTCGCCGACGAGTACGGCTCCGGCGAGCTCCGCCTCACCGTG
GAGCAGAACATCGTCCTCCCCAACGTCAAGAACGAGAAGGTGGAAGCTCTGCTCGCC
GAGCCGCTGCTGCAGAAGTTCTCGGCGCACCCGTCGCTGCTGATGAAGGGGCTGGTG
GCGTGCACGGGCAACCAGTTCTGCGGGCAGGCCATCATCGAGACCAAGGCGCGGGCG
CTGCAGGTGACGCGCGACGTGGAGGCGCGCGTGTCCGTGCCCAGGGCGGTGCGCATG
CACTGGACGGGGTGCCCCAACAGCTGCGCGCAGGTGCAGGTGGCCGACATCGGCTTC
ATGGGCTGCCTCACCAAGAACAGCAGCGGCAAGATCGTCGAGGCGGCGGACATCTTC
GTCGGCGGCCGCGTCGGCAGCGACTCGCACCTCACCGGGGTGTACAAGAAGGCGGTG
CCGTGCGAGGACCTGGTGCCCCTCGTCGCCGACCTCCTGGTGGAGCGGTTCGGGGCC
GTGCCCAGGGAGAGGGAGGAGGACGAGGAGTAG
SEQ ID NO:5 Amino acid sequence of tomato NiR MASFSIKFLAPSLPNPTRFSKSSIVKLNATPPQTVAAAGPPEVAAERLEPRVEEKDGYWILK 
EQFRQGINPQEKVKIEKEPMKLFMENGIEELAKIPIEEIDQSKLTKDDIDVRLKWLGLFHRR
KNQYGRFMMRLKLPNGVTTSAQTRYLASVIRKYGEEGCADITTRQNWQIRGVVLPDVPEI
LKGLEEVGLTSLQSGMDNVRNPVGNPLAGIDPEEIVDTRPYTNLLSQFITGNSRGNPAVSN
LPRKWNPCVVGSHDLYEHPHINDLAYMPAIKDGRFGFNLLVGGFFSAKRCDEAIPLDAWV
PADDVVPVCKAILEAFRDLGFRGNRQKCRMMWLIDELGVEGFRAEVVKRMPQQELERAS
PEDLVQKQWERRDYLGVHPQKQEGYSFIGLHIPVGRVQADDMDDLARLADEYGSGELRL
TVEQNIIIPNIENSKIDALLKEPILSKFSPDPPILMKGLVACTGNQFCGQAIIETKARSLKITEE
VQRQVSLTRPVRMHWTGCPNTCAQVQVADIGFMGCLTRDKDKKTVEGADVFLGGRIGS
DSHLGEVYKKAVPCDELVPLIVDLLIKNFGAVPREREETED
SEQ ID NO:6 Coding sequence of tomato SlNiR

ATGGCATCTTTTTCTATCAAATTTTTGGCACCTTCATTGCCAAATCCAACTAGATTTTCC
AAGAGTAGTATTGTCAAGCTCAATGCAACTCCGCCGCAGACAGTGGCTGCGGCGGGGC
CTCCAGAGGTTGCTGCTGAGAGACTAGAACCAAGAGTTGAGGAAAAAGATGGATATT
GGATACTAAAAGAGCAGTTTAGGCAAGGAATTAATCCTCAAGAGAAGGTGAAGATTGA
GAAGGAACCTATGAAGTTGTTCATGGAAAATGGTATTGAGGAGTTAGCTAAGATTCCAA
TTGAAGAGATAGATCAATCAAAGCTTACTAAGGATGACATTGATGTTAGGCTCAAGTGG
CTTGGCCTCTTCCATAGGAGAAAGAATCAATATGGGAGATTCATGATGAGGTTGAAACT
TCCAAATGGAGTAACAACAAGTGCTCAGACTCGATATTTGGCGAGTGTGATAAGGAAA
TATGGAGAGGAAGGATGTGCTGATATTACGACAAGGCAAAATTGGCAGATTCGTGGAG
TAGTGCTGCCTGATGTGCCTGAGATTCTAAAGGGACTTGAAGAAGTTGGCTTGACTAG
TTTGCAGAGTGGCATGGATAATGTCAGGAATCCAGTTGGAAATCCTCTGGCTGGAATTG
ATCCTGAAGAAATAGTTGACACAAGACCTTACACTAATTTGCTCTCCCAATTTATCACT
GGTAATTCACGAGGCAATCCGGCTGTTTCTAACTTGCCAAGGAAGTGGAATCCGTGTG
TAGTAGGGTCTCATGATCTTTATGAGCACCCTCATATCAATGATCTTGCATACATGCCTG
CCATAAAAGATGGACGATTTGGATTCAACCTGCTTGTGGGAGGGTTCTTCAGTGCCAA
AAGATGTGATGAGGCAATTCCTCTTGATGCATGGGTTCCAGCCGATGATGTTGTTCCGG
TTTGCAAGGCAATACTGGAAGCTTTTAGAGACCTTGGGTTCAGAGGGAACAGGCAGA
AGTGTAGAATGATGTGGTTGATCGATGAACTGGGTGTAGAAGGATTCAGGGCAGAGGT
CGTAAAGAGAATGCCTCAGCAAGAGCTAGAGAGAGCATCTCCGGAAGACTTGGTTCA
GAAACAATGGGAAAGAAGAGATTATCTTGGTGTACATCCACAGAAACAGGAAGGCTAT
AGCTTTATTGGTCTTCACATTCCAGTGGGTCGTGTCCAAGCAGACGACATGGATGATCT
AGCTCGTTTGGCTGATGAGTACGGCTCAGGAGAGCTACGGCTGACTGTGGAACAGAA
CATTATTATTCCCAACATTGAGAACTCAAAGATTGACGCACTGCTAAAAGAGCCTATTT
TGAGCAAATTTTCACCTGATCCACCTATTCTCATGAAAGGTTTAGTGGCTTGTACTGGTA
ACCAGTTTTGTGGACAAGCCATTATTGAAACGAAAGCTCGTTCCCTGAAGATCACCGA

AGAGGTTCAAAGGCAAGTATCTCTAACGAGGCCAGTAAGGATGCACTGGACAGGCTG
CCCAAATACGTGTGCACAAGTTCAAGTTGCAGACATTGGATTCATGGGATGCCTGACTA
GAGATAAAGACAAGAAGACTGTGGAAGGCGCCGATGTTTTCTTAGGAGGCAGAATAG
GGAGTGACTCACATTTGGGTGAAGTATACAAGAAGGCAGTTCCTTGTGATGAATTAGTA
CCACTTATTGTGGACTTACTTATTAAGAACTTTGGTGCAGTTCCACGAGAAAGAGAAG
AAACAGAAGAT

## Claims

1. A method of improving regeneration efficiency of a plant cell, comprising:
(a) overexpressing NiR in the plant cell, such as introducing an expression construct containing a coding nucleic acid sequence of NiR into the plant cell;
(b) regenerating into an intact plant from the plant cell.

2. A method of transforming at least one exogenous nucleic acid sequence of interest into a plant, comprising:
(a) overexpressing NiR in a cell of the plant, such as introducing an expression construct containing a coding nucleic acid sequence of NiR into the cell of the plant;
(b) introducing at least one expression construct containing at least one exogenous nucleic acid sequence of interest into the plant cell; and
(c) regenerating into an intact plant from the plant cell.

3. A method of gene editing in a plant, comprising:
(a) overexpressing NiR in a cell of the plant, such as introducing an expression construct containing a coding nucleic acid sequence of NiR into the cell of the plant;
(b) introducing at least one expression construct containing at least one exogenous nucleic acid sequence of interest into the plant cell, wherein the at least one exogenous nucleic acid sequence of interest encodes a component of the gene editing system;
(c) regenerating into an intact plant from the plant cell.

4. The method of claim 2 or 3, wherein the coding nucleic acid sequence of NiR and the at least one exogenous nucleic acid sequence of interest are present in the same expression construct.

5. The method of any one of claims 1-4, wherein the NiR contains the amino acid sequence shown in SEQ ID NO:1, 3 or 5.

6. The method of any one of claims 3-5, wherein the gene editing system is selected from a CRISPR system, TALEN, meganuclease and zinc-fingernuclease.

7. The method of claim 6, wherein the gene editing system is a CRISPR system, such as a base editing system.

8. The method of any one of claims 1-7, wherein the cell is selected from a protoplast cell, a callus cell, an immature embryo cell, and an explant cell.

9. The method of any one of claims 1-8, wherein the plant is selected from wheat, strawberry, rice, corn, soybean, sunflower, sorghum, rape, alfalfa, cotton, barley, millet, sugarcane, tomato, tobacco, cassava and potato.
